# EUROPEAN PATENT APPLICATION

(11) **EP 4 147 734 A1**
(43) Date of publication of application: **15.03.2023**
(21) Application number: 22195632.9
(22) Date of filing: 14.09.2022
(51) Int. Cl.: A61M 5/14, A61M 5/142

(54) **DUAL CHANNEL INFUSION PUMP FOR CONTINUOUS INFUSION**

(30) Priority: 14.09.2021 US 202163244170 P
(71) Applicant: Carefusion 303 Inc., San Diego, California 92130 (US)
(72) Inventor: Meng, Fanqing, San Diego, 92130 (US); Carroll, Derek Alan, San Diego, 92130 (US); Kimm, Daniel I., San Diego, 92130 (US); Weimer, Marc William, San Diego, 92130 (US)
(74) Representative: Epping - Hermann - Fischer

(57) **Abstract**

An infusion pump includes first and pumping segments configured to operate in parallel with each other, each pumping segment including a group of serially-aligned pumping elements configured to compress an elongated compressible channel loaded within the pumping segment. Each group of serially aligned pumping elements of the multiple pumping segments is caused to pump according to respective timing patterns offset from each other to deliver a first fluid from a first compressible channel loaded in the first pumping segment to a common delivery tubing while filling a second compressible channel loaded in the second pumping segment with a second fluid, and to deliver the second fluid from second compressible channel to the common delivery conduit while filling the first compressible channel using the first pumping segment.

## Description

### BACKGROUND

This application relates generally to a pumping mechanism for an infusion pump.

As a result of the ongoing need for improved health care, there is a continuous effort with regard to administering intravenous fluid to patients. As is well known, medication dispensers and infusion devices are used for infusion of predetermined amounts of medication into the body of a patient. Various types of medication dispensers employing different techniques for a variety of applications are known to exist. Some existing infusion devices employ a finger type pump unit having fingers which are moved in predetermined sequence to squeeze a feeding tube to infuse predetermined amounts of medication continuously, by way of pulsed boluses, into a patient. In many cases it is of critical importance to provide precisely controlled and consistent flow rates of intravenous fluid to patients. This need for more controlled IV flow rates is only partially fulfilled by the above-mentioned displacement pumps.

### SUMMARY

The subject technology provides a modified design of an infusion pump with a dual channel pumping segment array that changes pulse infusion to a continuous infusion. The subject technology provides a medication infusion device having two or more pumping segments which operate in parallel to alternately and collaboratively drive an intravenous (IV) tubing set so that one channel delivers a fluid to the patient while the other one completes the filling phase, simultaneously.

According to various implementations, an infusion system comprises an infusion device having multiple pumping segments configured to operate in parallel with each other, each pumping segment including a group of serially-aligned pumping elements configured to compress an elongated compressible channel loaded within the pumping segment; a processor configured to, when a respective compressible channel is loaded in each of the multiple pumping segments: operate each group of serially aligned pumping elements of the multiple pumping segments according to respective timing patterns offset from each other to deliver a first fluid from a first compressible channel loaded in a first pumping segment to a common delivery conduit while filling a second compressible channel loaded in a second pumping segment with a second fluid, and to deliver the second fluid from second compressible channel to the common delivery conduit while filling the first compressible channel using the first pumping segment.

In some implementations, the multiple pumping segments comprise a first pumping segment and a second pumping segment, the pumping elements of each of the first and second pumping segments comprising an upstream occluder, a downstream occluder, and a plunger, wherein delivering a fluid from a respective compressible channel comprises: filling the respective compressible channel while the upstream occluder is open and the downstream occluder is closed, closing the upstream occluder and opening the downstream occluder after the compressible channel is filled, and compressing the compressible channel using the plunger while the downstream occluder is opened, wherein the processor is further configured to: open the upstream occluder of the first pumping segment while the upstream occluder of the second pumping segment is closed or closing; and close the upstream occluder of the first pumping segment while the upstream occluder of the second pumping segment is open or opening.

Other aspects include corresponding methods, apparatuses, and computer program products for implementation of the foregoing features of the disclosed system.

According to various implementations, a machine-implemented method comprises receiving a first compressible channel into a first pumping segment of an infusion device, the first pumping segment comprising a first group of serially aligned pumping elements that operate collectively to delivery a first fluid from a first compressible channel when the first compressible channel is received into the first pumping segment; receiving, while the first compressible channel is received into the first pumping segment, a second compressible channel into a second pumping segment of the infusion device, the second pumping segment comprising a second group of serially aligned pumping elements that operate collectively to delivery a second fluid from a second compressible channel when the second compressible channel is received into the second pumping segment; and operating each group of serially aligned pumping elements of the first and second pumping segments according to respective timing patterns offset from each other to deliver the first fluid from the first compressible channel received in the first pumping segment to a common delivery conduit while filling the second compressible channel loaded in the second pumping segment with the second fluid, and to deliver the second fluid from second compressible channel to the common delivery conduit while filling the first compressible channel using the first pumping segment.

In some implementations, each of the first and second pumping segments comprise an upstream occluder, a downstream occluder, and a plunger, wherein delivering a fluid from a respective compressible channel comprises: filling the respective compressible channel while the upstream occluder is open and the downstream occluder is closed, closing the upstream occluder and opening the downstream occluder after the compressible channel is filled, and compressing the compressible channel using the plunger while the downstream occluder is opened, and wherein the machine-implemented method further comprises: opening the upstream occluder of the first pumping segment while the upstream occluder of the second pumping segment is closed or closing; and closing the upstream occluder of the first pumping segment while the upstream occluder of the second pumping segment is open or opening.

Other aspects include corresponding apparatuses, systems, and computer program products for implementation of the foregoing features of the disclosed method.

According to various implementations, an infusion pump comprises first and second pumping segments configured to operate in parallel with each other, each pumping segment including a group of serially-aligned pumping elements configured to compress an elongated compressible channel loaded within the pumping segment; and a processor configured to, when a respective compressible channel is loaded in each of the first and second pumping segments: cause each group of serially aligned pumping elements of the first and second pumping segments to pump according to respective timing patterns offset from each other to deliver a first fluid from a first compressible channel loaded in the first pumping segment to a common delivery conduit while filling a second compressible channel loaded in the second pumping segment with a second fluid, and to deliver the second fluid from second compressible channel to the common delivery conduit while filling the first compressible channel using the first pumping segment.

Other aspects include corresponding systems, methods, apparatuses, and computer program products for implementation of the foregoing features of the infusion pump.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

For a better understanding of the various described implementations, reference should be made to the Description of Implementations below, in conjunction with the following drawings. Like reference numerals refer to corresponding parts throughout the figures and description.
FIG. 1A depicts an example pumping mechanism of an infusion pump including two occluder valves, according to various aspects of the subject technology.
FIG. 1B depicts an example delivery volume pattern over time for the pumping mechanism of FIG. 1A.
FIG. 2 depicts an example dual channel infusion pump unit and a corresponding dual channel infusion set for providing continuous fluid infusion, according to various aspects of the subject technology.
FIG. 3 depicts a perspective view of an example dual channel infusion pump unit showing a dual channel infusion set in place within the infusion pump, according to various aspects of the subject technology.
FIG. 4A depicts an example dual channel pumping mechanism of a dual channel infusion pump, according to various aspects of the subject technology.
FIG. 4B depicts an example delivery volume pattern over time for the dual channel pumping mechanism of FIG. 4A.
FIGS. 5A and 5B depict example phase shifted delivery volume patterns over time for the dual channel pumping mechanism of FIG. 4A, according to various aspects of the subject technology.
FIG. 6 depicts an example process for providing continuous fluid infusion using a dual channel infusion pump, according to aspects of the subject technology.
FIG. 7 depicts an example diagram of an institutional patient care device of a healthcare organization, according to aspects of the subject technology.
FIG. 8 is a conceptual diagram illustrating an example electronic system for providing continuous fluid infusion using a dual channel infusion pump, according to aspects of the subject technology.

### DESCRIPTION

Reference will now be made to implementations, examples of which are illustrated in the accompanying drawings. In the following description, numerous specific details are set forth in order to provide an understanding of the various described implementations. However, it will be apparent to one of ordinary skill in the art that the various described implementations may be practiced without these specific details. In other instances, well-known methods, procedures, components, circuits, and networks have not been described in detail so as not to unnecessarily obscure aspects of the implementations.

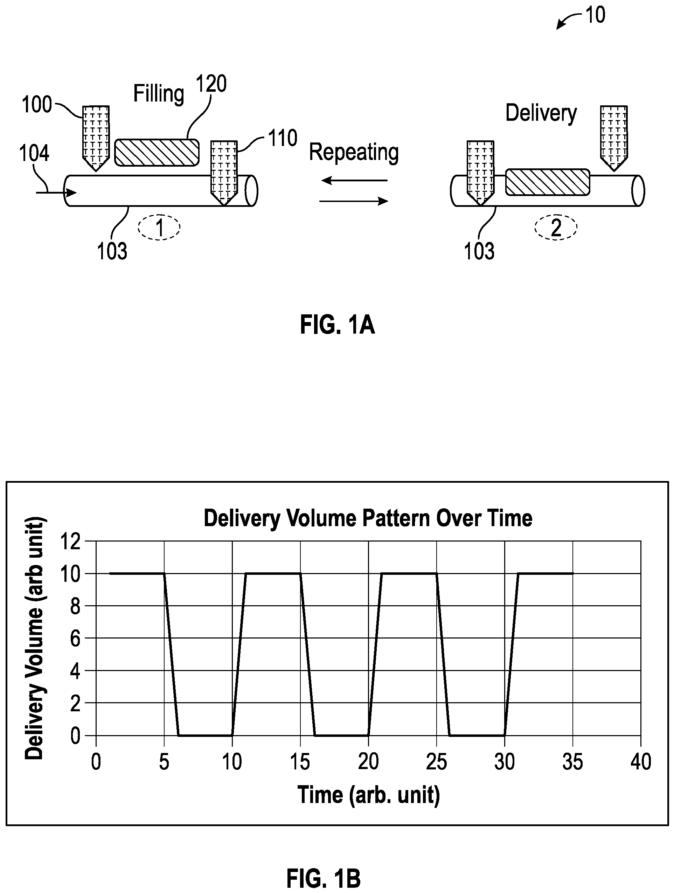

FIG. 1A depicts an example pumping mechanism 10 of an infusion device 12 including two occluder valves 100, 110, according to various aspects of the subject technology. A typical peristaltic medical pump for IV infusion delivery has two occluders, a first occluder 100 located upstream and a second occluder 110 located downstream, with a plunger 120 in between. The occluders and plunger coordinate with each other in programmable, sequential steps, controlled by a cam shaft to have two phases: 1) a filling phase, and 2) a delivery phase. The occluders move fluid in a tubing 103 by sequentially compressing the tubing, thereby causing a flow in a direction 104 according to the particular compression sequence of the occluders.

During the medication infusion process, in the filling phase, the upstream occluder 100 lifts to suck the medication into the tubing segment, which creates a pause, followed by the delivery phase to push the fluid out. These sequences can repeat through multiple cycles. To specify, when the plunger of a single plunger/tubing design is lifted from the tubing segment during the filling phase, there will be a disruption in the continuous infusion process. As a result of using this design, the medication delivery will behave with a pulse patten as shown in FIG. 1B.

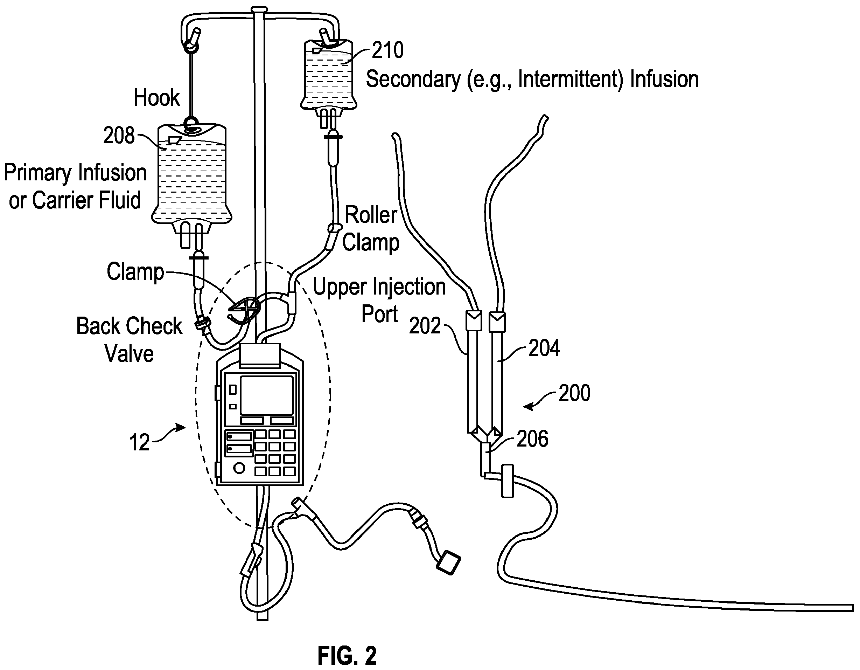

FIG. 2 depicts an example dual channel infusion pump unit 12 and a corresponding dual channel infusion set for providing continuous fluid infusion, according to various aspects of the subject technology. The subject technology provides a dual channel of pumping tubing segment array design to overcome the shortcomings of pulse infusion. By choosing two appropriate sets of occluders and plungers and linking them to the pump's mechanical cam shaft and motor, the medication delivery patten may be changed from a pulse infusion to a continuous and consistent infusion.

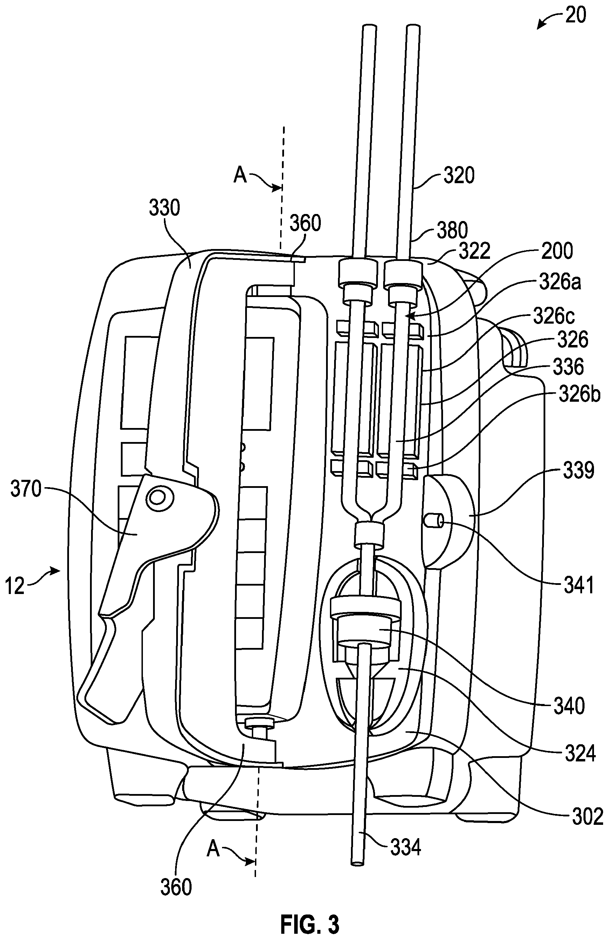

In this regard, the subject technology may include a dual channel infusion set 200. In the depicted example, the infusion set 200 includes two different elongated tubular segments 202, 204 that merge at a distal end 206 downstream (e.g., by way of a y-connector) to form a single intravenous (IV) line that may connect to a patient infusion site (not shown). Each tubing segment may be connected to an upstream delivery line, further connected to a respective fluid container 208, 210 (e.g., containing a medication or carrier fluid). Each tubing segment 202, 204 may be rigid or semi-rigid and configured to be installed or mounted within a dual channel infusion pump, as depicted in FIG. 3.

As will be described further, in a certain time interval, a first tubing segment 202 may be driven by pump mechanical parts to deliver the medication. In the meantime, a second tubing segment 204 may be recovering from its previous delivery phase to suck the fluid and move to the next measurement phase. To have a continuous infusion patten, the time duration in the delivery phase in the first channel may be equal to the time duration of the combination of the filling phase and measurement phase in the second channel and vice versa in the coming cycles alternatively.

FIG. 3 depicts a perspective view of an example dual channel infusion pump unit showing a dual channel infusion set in place within the infusion pump, according to various aspects of the subject technology. An infusion system for parenteral infusion of a medical fluid to a patient comprises a pump unit, a major part of which comprises a housing which accommodates, in manner known per se, a cam system (not shown) controlling a plurality of fingers of a peristaltic pumping mechanism, an electric motor and associated gearing, driving said cam mechanism, and further accommodates electronic control and processing circuitry for controlling such motor and processing signals from pressure sensors etc. provided on the unit. The pump unit, as shown, may also comprise an electronically operated display, an alarm light, an input keyboard or other manually operated controls, all in manner known per se.

As shown in FIG. 3, the infusion device may include a door 330 or face plate which may be opened to reveal the internal loading mechanism for the dual channel infusion set 200. Within the housing of the infusion device (e.g., behind the door or face place), the infusion device includes first and second pumping segments configured to operate in parallel with each other. Each pumping segment including a group of serially-aligned pumping elements configured to compress an elongated compressible channel of the infusion set 200, when loaded within the pumping segment. As will be described further, each pumping segment includes a group of serially-aligned pumping elements (e.g., occluders and/or pumping finger(s)) configured to compress the elongated compressible channel (e.g., an IV tubing segment) loaded within the pumping segment.

The infusion set 200 includes upper and lower sections 380 and 334 respectively of a plastics tubing, an intermediate section 336 of resiliently compressible tubing, for example of silicone rubber and, in some implementations, upper and/or lower fittings 338 and/or 340 via which each tubing section 336 may be connected respectively with a respective upper line 320 and with the lower line 334. In use, each upper line 320 extends upwardly to a source of the medical fluid to be administered whilst the lower line 334 extends from the infusion pump to an infusion needle or the like inserted into the patient. In use, the infusion set 200 is extended across the face or deck of the pump unit so that the fittings 338 and 340 are received in respective brackets 322 and 324 respectively and so that each tubing segment 202, 204 extends over a respective peristaltic assembly 326 as illustrated in FIG. 3. The infusion set 200 is fitted in place in this fashion whilst the door 330 is in the open position. After the infusion line has been so fitted, the door 330 may be moved to the closed position and is secured by a catch 37 which may include a lever mounted on the outer edge of the door.

The infusion device 12 includes a pump housing 302 and the multiple pumping segments 326 are retained by the pump housing of the infusion device. According to various aspects of the subject technology, the pump housing is configured to simultaneously receive the first and second compressible channels.

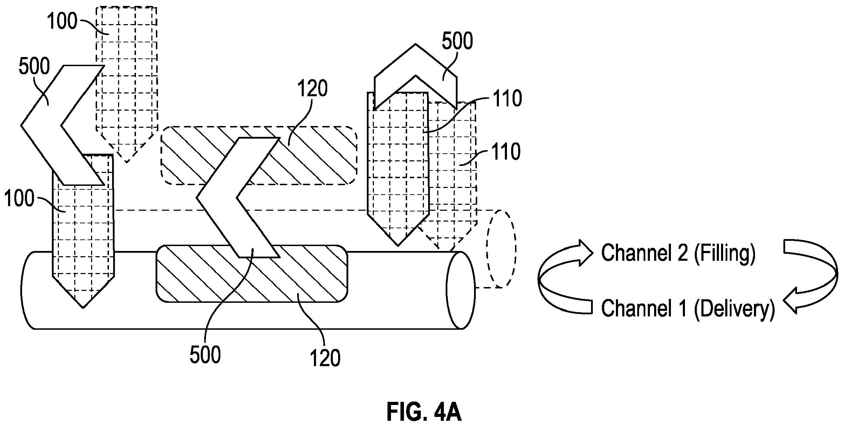

Each pumping segment 326 may include a peristaltic assembly with respective fingers that are moveable by a cam system (not shown) inwards and outwards from the face or deck of the pump to compress a respective tubing segment 202, 204 against a counter surface or anvil to propel fluid within the infusion line. According to various implementations, two cam systems operate the two depicted peristaltic assemblies independent of each other. A first cam system including one or more cams for operating the pumping elements 326a, 326b, 326c (see also FIG. 4A) of the first pumping segment, and a second cam system including one or more cams for operating the pumping elements 326a, 326b, 326c of the second pumping segment.

In order to make it easier to maintain sterile conditions, these fingers may be covered by a thin flexible membrane, (not shown), sealed at its edges with respect to the deck. The fingers of the peristaltic assembly 326 periodically press the flexible resilient tubing against the counter surface which may be configured on an opposite side, for example, on an inner portion of the door 330. In the example pump shown, each peristaltic assembly includes an upper occluder 326 a and a lower occluder 326 b which are of a relatively limited extent in the longitudinal direction of the infusion line, and an intermediate finger or pad 326 c, between the upper and lower fingers and which one or more fingers 326 c is extended or elongated in the longitudinal direction of the infusion line. In operation, assuming the fluid is to be propelled downwards, as viewed in FIGS. 1A and 4A and, along the infusion line, the peristaltic assembly performs a repeating cycle in which, with the intermediate pad 326 c spaced from the counter surface, the upper finger presses the flexible tube against the counter surface or anvil to close the tube at the location of the upper finger 326 a, the lower finger is then withdrawn from the counter surface to open the tube at the location of the lower finger 326 b, then the intermediate pad or finger 326 c is moved towards the counter surface to drive the fluid in the tube adjacent the intermediate pad 326 c downward along the tube, then the tube is pinched closed again between the lower finger 326 b and the counter surface, then the upper finger 326 a is withdrawn from the counter surface and the intermediate finger 326 c withdrawn from the counter surface to draw fresh fluid into the part of the tube adjacent the intermediate finger 326 c.

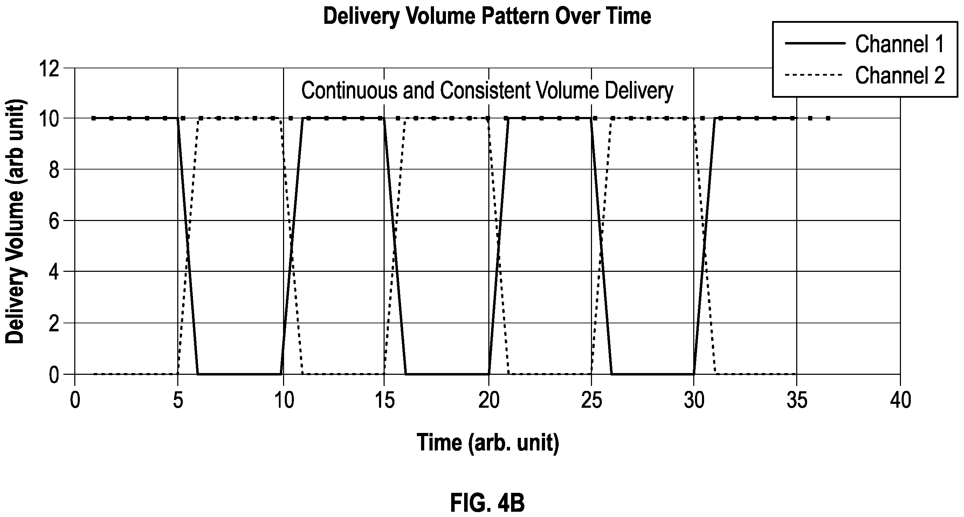

FIG. 4A depicts an example dual channel pumping mechanism of a dual channel infusion pump, according to various aspects of the subject technology. As described previously, the subject technology includes a dual channel of two pumping segments in parallel that interact with two groups of occluders and plungers to drive the tubing alternately and collaboratively in a programmable mechanical movement. In this manner, one channel delivers the fluid while the other one completes the filling phase simultaneously. The overall delivery pattern over time in the jointed downstream conduit is continuous. FIG. 4B depicts an example delivery volume pattern over time for the dual channel pumping mechanism of FIG. 4A.

Accordingly, multiple pumping segments (100, 101, 102) are configured to operate in parallel with each other. Each pumping segment includes a group of serially-aligned pumping elements (e.g., occluders and/or pumping finger(s)) configured to compress an elongated compressible channel (e.g., an IV tubing segment) loaded within the pumping segment. Each group of serially aligned pumping elements may be caused (e.g., by a processor) to pump according to respective timing patterns offset from each other to deliver a first fluid from a first compressible channel loaded in the first pumping segment to a common delivery conduit while filling a second compressible channel loaded in the second pumping segment with a second fluid, and to deliver the second fluid from second compressible channel to the common delivery conduit while filling the first compressible channel using the first pumping segment.

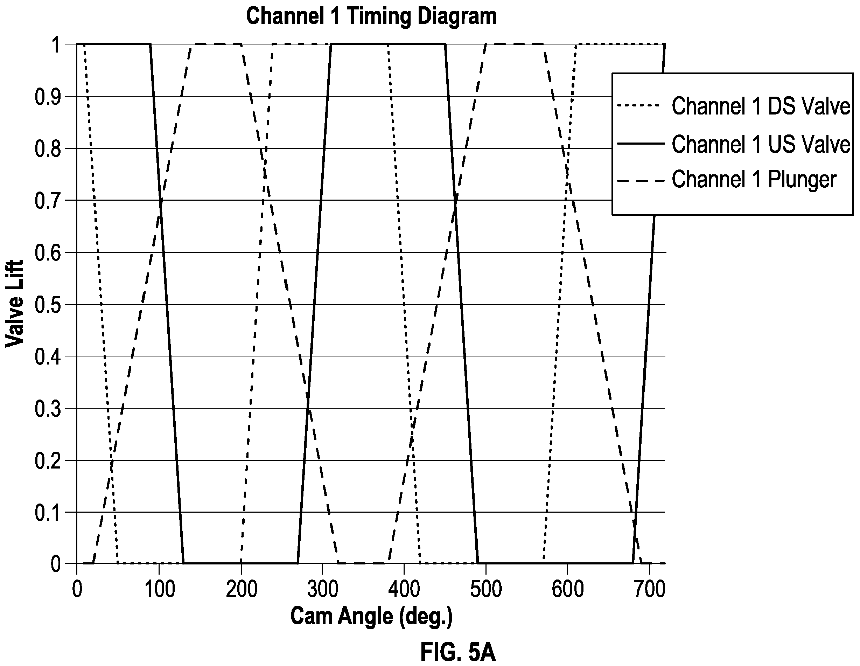

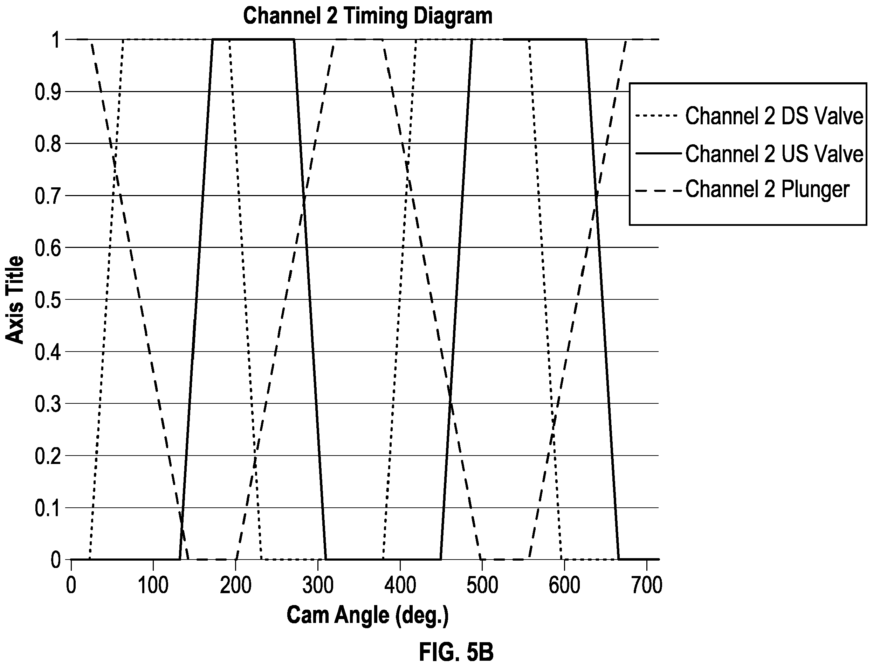

FIGS. 5A and 5B depict example phase shifted delivery volume patterns over time for the dual channel pumping mechanism of FIG. 4A, according to various aspects of the subject technology. In some implementations, a lever 500 can be linked between the two upstream occluders 100, the two downstream occluders 101, and the two plungers 120 respectively to have a balanced and optimized coordination between channels 1 and 2. The timing of channel 1 with regard to channel 2 may be biased, for example, by approx. 180 degrees of one cam revolution as shown in Fig. 5 to express the phase shift of downstream valve, upstream valve and plunger.

In some implementations, the two sets of occluders and plungers can be linked to a separate cam shaft/motor and can be programmed to change the way the pump is to deliver either a pulse infusion or continuous infusion on demand. For example, there may be some benefit of flushing catheters before and after use to prevent catheter occlusions. The pulsatile flushing with saline solution may be more efficient at clearing catheters of solid deposits than flushing the catheter with a single bolus. Accordingly, in some implementations, more overlap of flow between channels 1 and 2 can be programmed to provide optimal flow continuity during the "wrap around" where the pump moves from "delivery" to "refill".

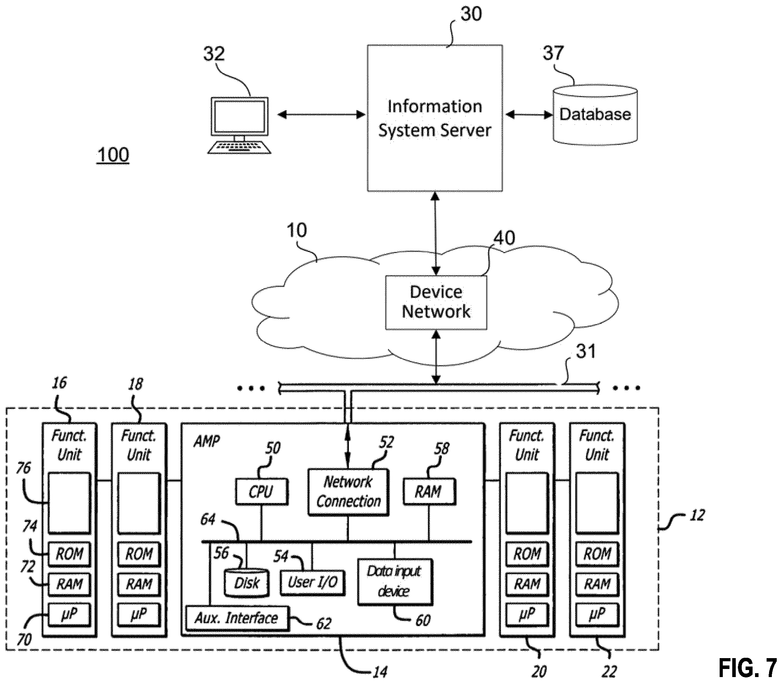

Some of mediations (e.g., chemotherapy, antibiotics, electrolytes) are administered (e.g., by way of intermittent infusions) through a secondary IV bag (Piggyback) attached to a Y-port below the pump. The Y-port may provide for a flushing, thus minimizing medication loss in the residual volume left behind in the tubing. In some implementations, the dual channel delivery pattern of FIGS. 4-5 may be accomplished by algorithm control of multiple pump modules (see FIG. 7) of a single pump. A split between secondary and primary infusion could be upstream of the pump. The primary and secondary infusate can be run separately to draw the medication from respective IV containers. One module may function as channel 1 and the other module as channel 2, with a control unit 14 (see FIG. 7) providing timing signals to each respective pump mechanism to provide a timed delivery volume, as shown in FIGS. 4B, 5A, and 5B. Additionally or in the alternative, the two types of infusate may be mixed precisely in a predetermined and controlled manner by way of programming the pumping mechanism to fire at specific intervals determined to proportionally delivery the medication from each container.

When connected appropriately, after the intermittent infusion is complete, the fluid from primary line may be infused to flush residual drug from the tubing. This would remove many constraints of the existing architecture and allow for a much smaller residual volume and reduce secondary clamp errors, connection errors and pressure differential errors.

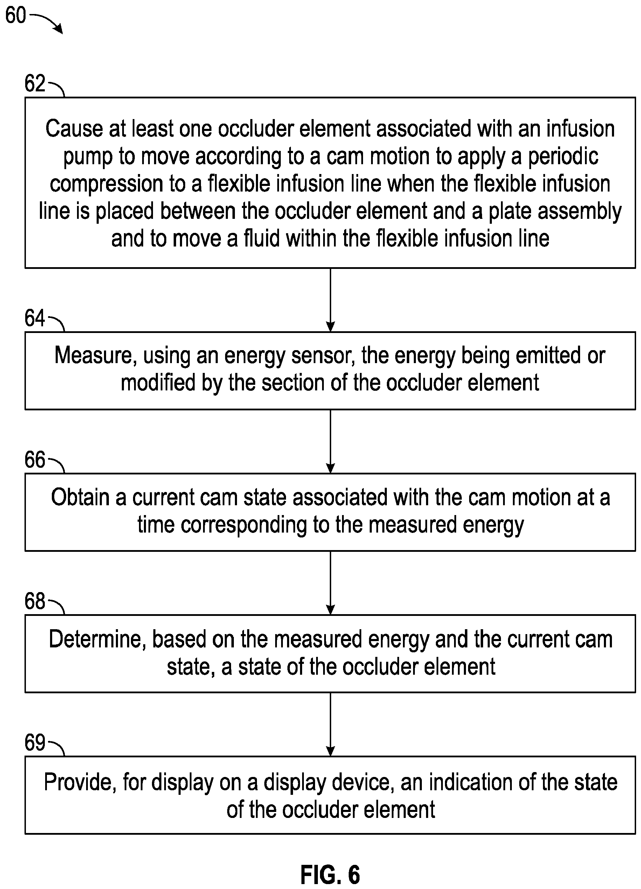

FIG. 6 depicts an example process for providing continuous fluid infusion using a dual channel infusion pump, according to aspects of the subject technology. For explanatory purposes, the various blocks of example process 60 are described herein with reference to FIGS. 1 through 5, and the components and/or processes described herein. The one or more of the blocks of process 60 may be implemented, for example, by one or more computing devices including, for example, within infusion device 12 of FIGS. 2, 3, and/or 7. In some implementations, one or more of the blocks may be implemented based on one or more machine learning algorithms. In some implementations, one or more of the blocks may be implemented apart from other blocks, and by one or more different processors or devices. Further for explanatory purposes, the blocks of example process 60 are described as occurring in serial, or linearly. However, multiple blocks of example process 60 may occur in parallel. In addition, the blocks of example process 60 need not be performed in the order shown and/or one or more of the blocks of example process 60 need not be performed.

In the depicted example, a first compressible channel is received into a first pumping segment of an infusion device (62). The first pumping segment 326 includes a first group of serially aligned pumping elements that operate collectively to delivery a first fluid from a first compressible channel when the first compressible channel is received into the first pumping segment. The pumping elements may include, for example, an upstream occluder 326a, 100, a downstream occluder 326b, 110, and a plunger 326c, 120. In this regard, delivering a fluid from a respective compressible channel may include, for example, filling the respective compressible channel while the upstream occluder is open and the downstream occluder is closed, closing the upstream occluder and opening the downstream occluder after the compressible channel is filled, and compressing the compressible channel using the plunger while the downstream occluder is opened.

While the first compressible channel is received into the first pumping segment, a second compressible channel is received into a second pumping segment of the infusion device (64). Similar to the first pumping segment, the second pumping segment includes a second group of serially aligned pumping elements that operate collectively to delivery a second fluid from a second compressible channel when the second compressible channel is received into the second pumping segment. The pumping elements may include, for example, an upstream occluder, a downstream occluder, and a plunger.

A processor operates each group of serially aligned pumping elements of the first and second pumping segments according to respective timing patterns offset from each other to deliver the first fluid from the first compressible channel received in the first pumping segment to a common delivery conduit while filling the second compressible channel loaded in the second pumping segment with the second fluid (66). Likewise, the pumping element groups are coordinated to deliver the second fluid from second compressible channel to the common delivery conduit while filling the first compressible channel using the first pumping segment. The coordination may include opening the upstream occluder of the first pumping segment while the upstream occluder of the second pumping segment is closed or closing, and closing the upstream occluder of the first pumping segment while the upstream occluder of the second pumping segment is open or opening.

A processor (or processors) associated with the infusion device causes the occluders to move according to a cam motion to apply a periodic compression to a flexible infusion line when the flexible infusion line is placed between the occluder element and a plate assembly, and to move a fluid within the flexible infusion line. According to various implementations, the infusion device 12 includes a pump housing 302, and the pump housing 302 houses the first and second pumping segments, as depicted in FIG. 3. Accordingly, example process 60 may further include simultaneously receiving the first and second compressible channels into the pump housing.

In some implementations, one or more cams are controlled to operate the pumping elements of the first pumping segment, and one or more cams are controlled to operate the pumping elements of the second pumping segment. The timing pattern for operating the first pumping segment maybe offset from the timing pattern for operating the second pumping segment based on a rotation of a first cam system being rotationally offset from a rotation of a second cam system by a predetermined number of degrees greater than zero (e.g., 30°).

Additionally or in the alternative, each upstream occluder, each downstream occluder, and each plunger of the first and second pumping segments may be joined together by respective levers to coordinate movement of the first and second pumping segments as a respective pumping element set. The lever may coordinate a motion of each pumping element of the respective pumping element set based on a motion of the other pumping element of the respective pumping element set. Each group of serially aligned pumping elements may be operated according to respective timing patterns comprises operating the groups of serially aligned pumping elements so that the respective deliveries of the first and second fluids are continuous but do not overlap.

As will be described further with regard to FIG. 7, infusion device may include a control unit 14 and first and second pump modules 16, 18, 20, 22 removably connected to the control unit 14 by way of respective plugin ports on the control unit. The first pump module may include the first pumping segment and the second pump module comprising the second pumping segment. In this regard, the control unit 14 may include a processor 50 that communicates with the first and second pump modules via electrical signals communicated through the respective plug in ports.

Many of the above-described devices, systems and methods, may also be implemented as software processes that are specified as a set of instructions recorded on a computer readable storage medium (also referred to as computer readable medium), and may be executed automatically (e.g., without user intervention). When these instructions are executed by one or more processing unit(s) (e.g., one or more processors, cores of processors, or other processing units), they cause the processing unit(s) to perform the actions indicated in the instructions. Examples of computer readable media include, but are not limited to, CD-ROMs, flash drives, RAM chips, hard drives, EPROMs, etc. The computer readable media does not include carrier waves and electronic signals passing wirelessly or over wired connections.

The term "software" is meant to include, where appropriate, firmware residing in read-only memory or applications stored in magnetic storage, which can be read into memory for processing by a processor. Also, in some implementations, multiple software aspects of the subject disclosure can be implemented as sub-parts of a larger program while remaining distinct software aspects of the subject disclosure. In some implementations, multiple software aspects can also be implemented as separate programs. Finally, any combination of separate programs that together implement a software aspect described here is within the scope of the subject disclosure. In some implementations, the software programs, when installed to operate on one or more electronic systems, define one or more specific machine implementations that execute and perform the operations of the software programs.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

FIG. 7 depicts an example diagram of an institutional patient care device 70 of a healthcare organization, according to aspects of the subject technology. In FIG. 7, a patient care device (or "medical device" generally) 12 is connected to a hospital network 10. The term patient care device (or "PCD") may be used interchangeably with the term patient care unit (or "PCU"), either which may include various ancillary medical devices such as an infusion pump, a vital signs monitor, a medication dispensing device (e.g., cabinet, tote), a medication preparation device, an automated dispensing device, a module coupled with one of the aforementioned (e.g., a syringe pump module configured to attach to an infusion pump), or other similar devices. As described previously, PCU/PCD 12 (and/or the infusion pump) functions as the previously described reporter 104.

Each device 12 may be connected to an internal healthcare network 10 by a transmission channel 31. Transmission channel 31 is any wired or wireless transmission channel, for example an 802.11 wireless local area network (LAN). In some implementations, network 10 also includes computer systems located in various departments throughout a hospital. For example, network 10 of FIG. 1 optionally includes computer systems associated with an admissions department, a billing department, a biomedical engineering department, a clinical laboratory, a central supply department, one or more unit station computers and/or a medical decision support system. As described further below, network 10 may include discrete subnetworks. In the depicted example, network 10 includes a device network 40 by which patient care devices 12 (and other devices) communicate in accordance with normal operations.

Additionally, institutional patient care system 70 may incorporate a separate information system server 30. According to various implementations, server 30 and/or database 37 may incorporate, function as, or include a remote records system configured to store electronic medication administration records (eMAR) for patients admitted or cared for within the hospital organization. Although the information system server 30 is shown as a separate server, the functions and programming of the information system server 30 may be incorporated into another computer, if such is desired by engineers designing the institution's information system. Institutional patient care system 100 may further include one or multiple device terminals 32 for connecting and communicating with information system server 30. Device terminals 32 may include personal computers, personal data assistances, mobile devices such as laptops, tablet computers, augmented reality devices, or smartphones, configured with software for communications with information system server 30 via network 10.

Patient care device 12 comprises a system for providing patient care, such as for providing an infusion of medication to a patient. Patient care device 12 may include or incorporate an infusion pump, a physiological monitor (e.g., heart rate, blood pressure, ECG, EEG, pulse oximeter, and other patient monitors), therapy device, and other drug delivery device. In the depicted example, patient care device 12 comprises a control unit 14, also referred to as interface unit 14 or docking station, connected to one or more functional modules 16, 18, 20, 22. Interface unit 14 includes a central processing unit (CPU) 50 connected to a memory, for example, random access memory (RAM) 58, and one or more interface devices such as user interface device 54, a coded data input device 60, a network connection 52, and an auxiliary interface 62 for communicating with additional modules or devices. Interface unit 14 also, although not necessarily, includes a main non-volatile storage unit 56, such as a hard disk drive or non-volatile flash memory, for storing software and data and one or more internal buses 64 for interconnecting the aforementioned elements.

In various implementations, user interface device 54 is a touch screen for displaying information to a user and allowing a user to input information by touching defined areas of the screen. Additionally, or in the alternative, user interface device 54 could include any means for displaying and inputting information, such as a monitor, a printer, a keyboard, softkeys, a mouse, a track ball and/or a light pen. Data input device 60 may be a bar code reader capable of scanning and interpreting data printed in bar coded format. Additionally or in the alternative, data input device 60 can be any device for entering coded data into a computer, such as a device(s) for reading a magnetic strips, radio-frequency identification (RFID) devices whereby digital data encoded in RFID tags or smart labels (defined below) are captured by the reader 60 via radio waves, PCMCIA smart cards, radio frequency cards, memory sticks, CDs, DVDs, or any other analog or digital storage media. Other examples of data input device 60 include a voice activation or recognition device or a portable personal data assistant (PDA). Depending upon the types of interface devices used, user interface device 54 and data input device 60 may be the same device. Although data input device 60 is shown in FIG. 1 to be disposed within interface unit 14, it is recognized that data input device 60 may be integral within pharmacy system 34 or located externally and communicating with pharmacy system 34 through an RS-232 serial interface or any other appropriate communication means. Auxiliary interface 62 may be an RS-232 communications interface, however any other means for communicating with a peripheral device such as a printer, patient monitor, infusion pump or other medical device may be used without departing from the subject technology. Additionally, data input device 60 may be a separate functional module, such as modules 16, 18, 20 and 22, and configured to communicate with controller 14, or any other system on the network, using suitable programming and communication protocols.

Network connection 52 may be a wired or wireless connection, such as by Ethernet, WiFi, BLUETOOTH, an integrated service digital network (ISDN) connection, a digital subscriber line (DSL) modem or a cable modem. Any direct or indirect network connection may be used, including, but not limited to a telephone modem, an MIB system, an RS232 interface, an auxiliary interface, an optical link, an infrared link, a radio frequency link, a microwave link or a WLANS connection or other wireless connection.

Functional modules 16, 18, 20, 22 are any devices for providing care to a patient or for monitoring patient condition. As shown in FIG. 7, at least one of functional modules 16, 18, 20, 22 may be an infusion pump module such as an intravenous infusion pump for delivering medication or other fluid to a patient. For the purposes of this discussion, functional module 16 is an infusion pump module, and includes the forgoing pumping mechanism 10 including occluder valves. Each of functional modules 18, 20, 22 may be any patient treatment or monitoring device including, but not limited to, an infusion pump, a syringe pump, a PCA pump, an epidural pump, an enteral pump, a blood pressure monitor, a pulse oximeter, an EKG monitor, an EEG monitor, a heart rate monitor or an intracranial pressure monitor or the like. Functional module 18, 20 and/or 22 may be a printer, scanner, bar code reader or any other peripheral input, output or input/output device.

Each functional module 16, 18, 20, 22 may communicate directly or indirectly with interface unit 14, with interface unit 14 providing overall monitoring and control of device 12. Functional modules 16, 18, 20, 22 may be connected physically and electronically in serial fashion to one or both ends of interface unit 14 as shown in FIG. 7. However, it is recognized that there are other means for connecting functional modules with the interface unit that may be utilized without departing from the subject technology. It will also be appreciated that devices such as pumps or patient monitoring devices that provide sufficient programmability and connectivity may be capable of operating as stand-alone devices and may communicate directly with the network without connected through a separate interface unit or control unit 14. As described above, additional medical devices or peripheral devices may be connected to patient care device 12 through one or more auxiliary interfaces 62.

Each functional module 16, 18, 20, 22 may include module-specific components 76, a microprocessor 70, a volatile memory 72 and a nonvolatile memory 74 for storing information and performing the functions and/or operations described herein. It should be noted that while four functional modules are shown in FIG. 7, any number of devices may be connected directly or indirectly to central controller 14. The number and type of functional modules described herein are intended to be illustrative, and in no way limit the scope of the subject technology. Module-specific components 76 include any components necessary for operation of a particular module, such as a pumping mechanism for infusion pump module 16.

While each functional module may be capable of a least some level of independent operation, interface unit 14 monitors and controls overall operation of device 12. For example, as will be described in more detail below, interface unit 14 provides programming instructions to the functional modules 16, 18, 20, 22 and monitors the status of each module.

Patient care device 12 is capable of operating in several different modes, or personalities, with each personality defined by a configuration database. The configuration database may be a database 56 internal to patient care device, or an external database 37. A particular configuration database is selected based, at least in part, by patient-specific information such as patient location, age, physical characteristics, or medical characteristics. Medical characteristics include, but are not limited to, patient diagnosis, treatment prescription, medical history, medical records, patient care provider identification, physiological characteristics or psychological characteristics. As used herein, patient-specific information also includes care provider information (e.g., physician identification) or a patient care device's 10 location in the hospital or hospital computer network. Patient care information may be entered through interface device 52, 54, 60 or 62, and may originate from anywhere in network 10, such as, for example, from a pharmacy server, admissions server, laboratory server, and the like.

Medical devices incorporating aspects of the subject technology may be equipped with a Network Interface Module (NIM), allowing the medical device to participate as a node in a network. While for purposes of clarity the subject technology will be described as operating in an Ethernet network environment using the Internet Protocol (IP), it is understood that concepts of the subject technology are equally applicable in other network environments, and such environments are intended to be within the scope of the subject technology.

Data to and from the various data sources can be converted into network-compatible data with existing technology, and movement of the information between the medical device and network can be accomplished by a variety of means. For example, patient care device 12 and network 10 may communicate via automated interaction, manual interaction or a combination of both automated and manual interaction. Automated interaction may be continuous or intermittent and may occur through direct network connection 54 (as shown in FIG. 1), or through RS232 links, MIB systems, RF links such as BLUETOOTH, IR links, WLANS, digital cable systems, telephone modems or other wired or wireless communication means. Manual interaction between patient care device 12 and network 10 involves physically transferring, intermittently or periodically, data between systems using, for example, user interface device 54, coded data input device 60, bar codes, computer disks, portable data assistants, memory cards, or any other media for storing data. The communication means in various aspects is bidirectional with access to data from as many points of the distributed data sources as possible. Decision-making can occur at a variety of places within network 10. For example, and not by way of limitation, decisions can be made in HIS server 30, decision support 48, remote data server 49, hospital department or unit stations 46, or within patient care device 12 itself.

All direct communications with medical devices operating on a network in accordance with the subject technology may be performed through information system server 30, known as the remote data server (RDS). In accordance with aspects of the subject technology, network interface modules incorporated into medical devices such as, for example, infusion pumps or vital signs measurement devices, ignore all network traffic that does not originate from an authenticated RDS. The primary responsibilities of the RDS of the subject technology are to track the location and status of all networked medical devices, and maintain open communication.

With further reference to FIG. 7, an infusion device, as used herein, may be a patient care device 12, interface control unit 14, or a module 16, 18, 20, 22. According to various implementations, the infusion device includes a pump and a control unit. The control unit 14 is configured to provide, using the pump, an intravenous infusion of a medication to a current patient, and display on a display screen, while the infusion is being provided by the pump, a representation of a status of the intravenous infusion.

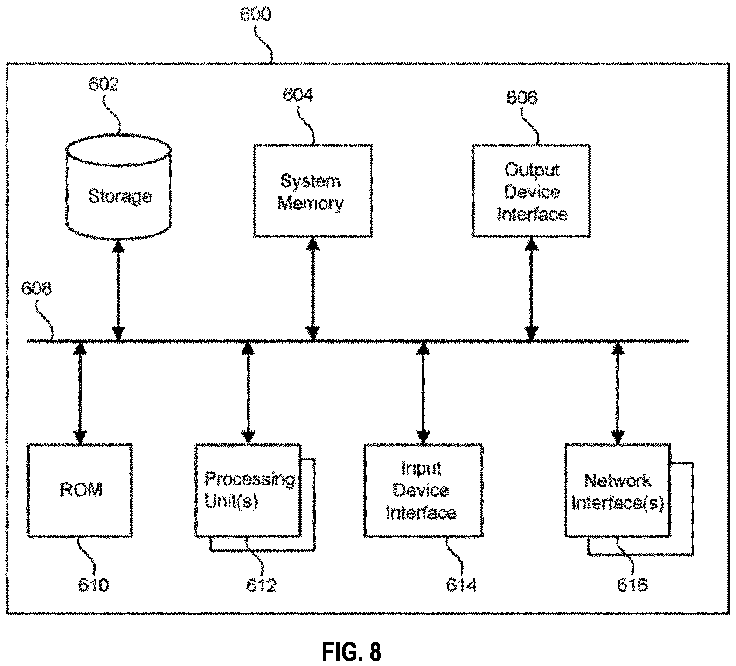

FIG. 8 is a conceptual diagram illustrating an example electronic system 600 for providing continuous fluid infusion using a dual channel infusion pump, according to aspects of the subject technology. Electronic system 600 may be a computing device for execution of software associated with one or more components and processes provided by FIGS. 1 to 7, including but not limited to controller 406, or computing hardware within pump 401 or system 330. Electronic system 600 may be representative of a device used in connection or combination with the disclosure regarding FIGS. 1 to 7. In this regard, electronic system 600 may be a personal computer or a mobile device such as a smartphone, tablet computer, laptop, PDA, an augmented reality device, a wearable such as a watch or band or glasses, or combination thereof, or other touch screen or television with one or more processors embedded therein or coupled thereto, or any other sort of computer-related electronic device having network connectivity.

Electronic system 600 may include various types of computer readable media and interfaces for various other types of computer readable media. In the depicted example, electronic system 600 includes a bus 608, processing unit(s) 612, a system memory 604, a read-only memory (ROM) 610, a permanent storage device 602, an input device interface 614, an output device interface 606, and one or more network interfaces 616. In some implementations, electronic system 600 may include or be integrated with other computing devices or circuitry for operation of the various components and processes previously described.

Bus 608 collectively represents all system, peripheral, and chipset buses that communicatively connect the numerous internal devices of electronic system 600. For instance, bus 608 communicatively connects processing unit(s) 612 with ROM 610, system memory 604, and permanent storage device 602.

From these various memory units, processing unit(s) 612 retrieves instructions to execute and data to process, in order to execute the processes of the subject disclosure. The processing unit(s) can be a single processor or a multi-core processor in different implementations.

ROM 610 stores static data and instructions that are needed by processing unit(s) 612 and other modules of the electronic system. Permanent storage device 602, on the other hand, is a read-and-write memory device. This device is a non-volatile memory unit that stores instructions and data even when electronic system 600 is off. Some implementations of the subject disclosure use a mass-storage device (such as a magnetic or optical disk and its corresponding disk drive) as permanent storage device 602.

Other implementations use a removable storage device (such as a floppy disk, flash drive, and its corresponding disk drive) as permanent storage device 602. Like permanent storage device 602, system memory 604 is a read-and-write memory device. However, unlike storage device 602, system memory 604 is a volatile read-and-write memory, such as random access memory. System memory 604 stores some of the instructions and data that the processor needs at runtime. In some implementations, the processes of the subject disclosure are stored in system memory 604, permanent storage device 602, and/or ROM 610. From these various memory units, processing unit(s) 612 retrieves instructions to execute and data to process in order to execute the processes of some implementations.

Bus 608 also connects to input and output device interfaces 614 and 606. Input device interface 614 enables the user to communicate information and select commands to the electronic system. Input devices used with input device interface 614 include, e.g., alphanumeric keyboards and pointing devices (also called "cursor control devices"). Output device interfaces 606 enables, e.g., the display of images generated by the electronic system 600. Output devices used with output device interface 606 include, e.g., printers and display devices, such as cathode ray tubes (CRT) or liquid crystal displays (LCD). Some implementations include devices such as a touchscreen that functions as both input and output devices.

Also, as shown in FIG. 8, bus 608 also couples electronic system 600 to a network (not shown) through network interfaces 616. Network interfaces 616 may include, e.g., a wireless access point (e.g., Bluetooth or WiFi) or radio circuitry for connecting to a wireless access point. Network interfaces 616 may also include hardware (e.g., Ethernet hardware) for connecting the computer to a part of a network of computers such as a local area network ("LAN"), a wide area network ("WAN"), wireless LAN, or an Intranet, or a network of networks, such as the Internet. Any or all components of electronic system 600 can be used in conjunction with the subject disclosure.

These functions described above can be implemented in computer software, firmware, or hardware. The techniques can be implemented using one or more computer program products. Programmable processors and computers can be included in or packaged as mobile devices. The processes and logic flows can be performed by one or more programmable processors and by one or more programmable logic circuitry. General and special purpose computing devices and storage devices can be interconnected through communication networks.

Some implementations include electronic components, such as microprocessors, storage and memory that store computer program instructions in a machine-readable or computer-readable medium (also referred to as computer-readable storage media, machine-readable media, or machine-readable storage media). Some examples of such computer-readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray^{®} discs, ultra density optical discs, any other optical or magnetic media, and floppy disks. The computer-readable media can store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

While the above discussion primarily refers to microprocessor or multi-core processors that execute software, some implementations are performed by one or more integrated circuits, such as application specific integrated circuits (ASICs) or field programmable gate arrays (FPGAs).

In some implementations, such integrated circuits execute instructions that are stored on the circuit itself.

As used in this specification and any claims of this application, the terms "computer", "server", "processor", and "memory" all refer to electronic or other technological devices. These terms exclude people or groups of people. For the purposes of the specification, the terms display or displaying means displaying on an electronic device. As used in this specification and any claims of this application, the terms "computer readable medium" and "computer readable media" are entirely restricted to tangible, physical objects that store information in a form that is readable by a computer. These terms exclude any wireless signals, wired download signals, and any other ephemeral signals.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; e.g., feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; e.g., by sending web pages to a web browser on a user's client device in response to requests received from the web browser.

Implementations of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

The computing system can include clients and servers. A client and server are generally remote from each other and may interact through a communication network. The relationship of client and server arises by virtue of computer programs running on the respective computers and having a client-server relationship to each other. In some implementations, a server transmits data (e.g., an HTML page) to a client device (e.g., for purposes of displaying data to and receiving user input from a user interacting with the client device). Data generated at the client device (e.g., a result of the user interaction) can be received from the client device at the server.

Those of skill in the art would appreciate that the various illustrative blocks, modules, elements, components, methods, and algorithms described herein may be implemented as electronic hardware, computer software, or combinations of both. To illustrate this interchangeability of hardware and software, various illustrative blocks, modules, elements, components, methods, and algorithms have been described above generally in terms of their functionality. Whether such functionality is implemented as hardware or software depends upon the particular application and design constraints imposed on the overall system. The described functionality may be implemented in varying ways for each particular application. Various components and blocks may be arranged differently (e.g., arranged in a different order, or partitioned in a different way) all without departing from the scope of the subject technology.

Illustration of Subject Technology as Clauses:
Various examples of aspects of the disclosure are described as numbered clauses (1, 2, 3, etc.) for convenience. These are provided as examples, and do not limit the subject technology. Identifications of the figures and reference numbers are provided below merely as examples and for illustrative purposes, and the clauses are not limited by those identifications.

Clause 1. An infusion system, comprising: an infusion device having multiple pumping segments configured to operate in parallel with each other, each pumping segment including a group of serially-aligned pumping elements configured to compress an elongated compressible channel loaded within the pumping segment; and a processor configured to, when a respective compressible channel is loaded in each of the multiple pumping segments: operate each group of serially-aligned pumping elements of the multiple pumping segments according to respective timing patterns offset from each other to deliver a first fluid from a first compressible channel loaded in a first pumping segment to a common delivery conduit while filling a second compressible channel loaded in a second pumping segment with a second fluid, and to deliver the second fluid from second compressible channel to the common delivery conduit while filling the first compressible channel using the first pumping segment.

Clause 2. The infusion system of Clause 1, wherein the multiple pumping segments comprise a first pumping segment and a second pumping segment, the pumping elements of each of the first and second pumping segments comprising an upstream occluder, a downstream occluder, and a plunger, wherein delivering a fluid from a respective compressible channel comprises: filling the respective compressible channel while the upstream occluder is open and the downstream occluder is closed, closing the upstream occluder and opening the downstream occluder after the respective compressible channel is filled, and compressing the respective compressible channel using the plunger while the downstream occluder is opened, wherein the processor is further configured to: open the upstream occluder of the first pumping segment while the upstream occluder of the second pumping segment is closed or closing; and close the upstream occluder of the first pumping segment while the upstream occluder of the second pumping segment is open or opening.

Clause 3. The infusion system of Clause 2, wherein the infusion device includes a pump housing and the multiple pumping segments are retained by the pump housing of the infusion device, wherein the pump housing is configured to simultaneously receive the first and second compressible channels.

Clause 4. The infusion system of Clause 3, wherein the infusion device comprises: a first cam system including one or more cams for operating the pumping elements of the first pumping segment; and a second cam system including one or more cams for operating the pumping elements of the second pumping segment.

Clause 5. The infusion system of Clause 3 or Clause 4, wherein each upstream occluder, each downstream occluder, and each plunger of the first and second pumping segments are joined together by respective levers to coordinate movement of the first and second pumping segments as a respective pumping element set, a respective lever coordinating a motion of each pumping element of the respective pumping element set based on a motion of the other pumping element of the respective pumping element set.

Clause 6. The infusion system of any one of Clauses 3 through 5, wherein the timing pattern for operating the first pumping segment is offset from the timing pattern for operating the second pumping segment based on a rotation of a first cam system being rotationally offset from a rotation of a second cam system by a predetermined number of degrees greater than zero.

Clause 7. The infusion system of any one of Clauses 1 through 6, wherein the infusion device comprises a control unit and first and second pump modules removably connected to the control unit by way of respective plugin ports on the control unit, the first pump module comprising the first pumping segment and the second pump module comprising the second pumping segment, wherein the control unit comprises the processor and the processor communicates with the first and second pump modules via electrical signals communicated through the respective plugin ports.

Clause 8. The infusion system of any one of Clauses 1 through 7, further comprising: a dual channel infusion set comprising the first compressible channel and the second compressible channel each joining together at a downstream junction for fluidic communication with the common delivery conduit, wherein the first compressible channel and the second compressible channel each include an upstream connector for connecting to a different fluid source.

Clause 9. The infusion system of any one of Clauses 1 through 8, wherein the common delivery conduit is an intravenous tubing connected to a patient.

Clause 10. The infusion system of Clause 9, wherein operating each group of serially aligned pumping elements according to respective timing patterns comprises operating the groups of serially aligned pumping elements so that the respective deliveries of the first and second fluids are continuous but do not overlap.

Clause 11. A machine-implemented method, comprising: receiving a first compressible channel into a first pumping segment of an infusion device, the first pumping segment comprising a first group of serially-aligned pumping elements that operate collectively to delivery a first fluid from a first compressible channel when the first compressible channel is received into the first pumping segment; receiving, while the first compressible channel is received into the first pumping segment, a second compressible channel into a second pumping segment of the infusion device, the second pumping segment comprising a second group of serially-aligned pumping elements that operate collectively to delivery a second fluid from a second compressible channel when the second compressible channel is received into the second pumping segment; and operating each group of serially-aligned pumping elements of the first and second pumping segments according to respective timing patterns offset from each other to deliver the first fluid from the first compressible channel received in the first pumping segment to a common delivery conduit while filling the second compressible channel loaded in the second pumping segment with the second fluid, and to deliver the second fluid from second compressible channel to the common delivery conduit while filling the first compressible channel using the first pumping segment.

Clause 12. The machine-implemented method of Clause 11, wherein each of the first and second pumping segments comprise an upstream occluder, a downstream occluder, and a plunger, wherein delivering a fluid from a respective compressible channel comprises: filling the respective compressible channel while the upstream occluder is open and the downstream occluder is closed, closing the upstream occluder and opening the downstream occluder after the respective compressible channel is filled, and compressing the respective compressible channel using the plunger while the downstream occluder is opened, wherein the machine-implemented method further comprises: opening the upstream occluder of the first pumping segment while the upstream occluder of the second pumping segment is closed or closing; and closing the upstream occluder of the first pumping segment while the upstream occluder of the second pumping segment is open or opening.

Clause 13. The machine-implemented method of Clause 12, wherein the timing pattern for operating the first pumping segment is offset from the timing pattern for operating the second pumping segment based on a rotation of a first cam system being rotationally offset from a rotation of a second cam system by a predetermined number of degrees greater than zero.

Clause 14. The machine-implemented method of Clause 12 or Clause 13, wherein operating each group of serially-aligned pumping elements according to respective timing patterns comprises operating the groups of serially-aligned pumping elements so that the respective deliveries of the first and second fluids are continuous but do not overlap.

Clause 15. The machine-implemented method of any one of Clauses 12 through 14, wherein the infusion device includes a pump housing, the pump housing comprising the first and second pumping segments, the method further comprising: simultaneously receiving the first and second compressible channels into the pump housing.

Clause 16. The machine-implemented method of any one of Clauses 12 through 15, further comprising: causing one or more cams to operate the pumping elements of the first pumping segment; and causing one or more second cams to operate the pumping elements of the second pumping segment.

Clause 17. The machine-implemented method of any one of Clauses 12 through 16, wherein each upstream occluder, each downstream occluder, and each plunger of the first and second pumping segments are joined together by respective levers to coordinate movement of the first and second pumping segments as a respective pumping element set, the method further comprising: a respective lever coordinating a motion of each pumping element of the respective pumping element set based on a motion of the other pumping element of the respective pumping element set.

Clause 18. The machine-implemented method of any one of Clauses 11 through 17, wherein the infusion device comprises a control unit and first and second pump modules removably connected to the control unit by way of respective plugin ports on the control unit, the first pump module comprising the first pumping segment and the second pump module comprising the second pumping segment, wherein the control unit comprises a processor and the processor communicates with the first and second pump modules via electrical signals communicated through the respective plugin ports.

Clause 19. The machine-implemented method of any one of Clauses 11 through 18, wherein the common delivery conduit is an intravenous tubing connected to a patient.

Clause 20. An infusion pump, comprising: first and second pumping segments configured to operate in parallel with each other, each pumping segment including a group of serially-aligned pumping elements configured to compress an elongated compressible channel loaded within the pumping segment; and a processor configured to, when a respective compressible channel is loaded in each of the first and second pumping segments: cause each group of serially-aligned pumping elements of the first and second pumping segments to pump according to respective timing patterns offset from each other to deliver a first fluid from a first compressible channel loaded in the first pumping segment to a common delivery conduit while filling a second compressible channel loaded in the second pumping segment with a second fluid, and to deliver the second fluid from second compressible channel to the common delivery conduit while filling the first compressible channel using the first pumping segment.

Further Consideration:
In some embodiments, any of the clauses herein may depend from any one of the independent clauses or any one of the dependent clauses. In one aspect, any of the clauses (e.g., dependent or independent clauses) may be combined with any other one or more clauses (e.g., dependent or independent clauses). In one aspect, a claim may include some or all of the words (e.g., steps, operations, means or components) recited in a clause, a sentence, a phrase or a paragraph. In one aspect, a claim may include some or all of the words recited in one or more clauses, sentences, phrases or paragraphs. In one aspect, some of the words in each of the clauses, sentences, phrases or paragraphs may be removed. In one aspect, additional words or elements may be added to a clause, a sentence, a phrase or a paragraph. In one aspect, the subject technology may be implemented without utilizing some of the components, elements, functions or operations described herein. In one aspect, the subject technology may be implemented utilizing additional components, elements, functions or operations.

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

The previous description is provided to enable any person skilled in the art to practice the various aspects described herein. The previous description provides various examples of the subject technology, and the subject technology is not limited to these examples. Various modifications to these aspects will be readily apparent to those skilled in the art, and the generic principles defined herein may be applied to other aspects. Thus, the claims are not intended to be limited to the aspects shown herein, but is to be accorded the full scope consistent with the language claims, wherein reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention described herein.

The term website, as used herein, may include any aspect of a website, including one or more web pages, one or more servers used to host or store web related content, etc. Accordingly, the term website may be used interchangeably with the terms web page and server. The predicate words "configured to", "operable to", and "programmed to" do not imply any particular tangible or intangible modification of a subject, but, rather, are intended to be used interchangeably. For example, a processor configured to monitor and control an operation or a component, may also mean the processor being programmed to monitor and control the operation or the processor being operable to monitor and control the operation. Likewise, a processor configured to execute code can be construed as a processor programmed to execute code or operable to execute code.

The term automatic, as used herein, may include performance by a computer or machine without user intervention; for example, by instructions responsive to a predicate action by the computer or machine or other initiation mechanism. The word "example" is used herein to mean "serving as an example or illustration." Any aspect or design described herein as "example" is not necessarily to be construed as preferred or advantageous over other aspects or designs.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "implementation" does not imply that such implementation is essential to the subject technology or that such implementation applies to all configurations of the subject technology. A disclosure relating to an implementation may apply to all implementations, or one or more implementations. An embodiment may provide one or more examples. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such as a "configuration" may refer to one or more configurations and vice versa.

## Claims

1. An infusion system, comprising:
an infusion device having multiple pumping segments configured to operate in parallel with each other, each pumping segment including a group of serially-aligned pumping elements configured to compress an elongated compressible channel loaded within the pumping segment;
a processor configured to, when a respective compressible channel is loaded in each of the multiple pumping segments:
operate each group of serially-aligned pumping elements of the multiple pumping segments according to respective timing patterns offset from each other to deliver a first fluid from a first compressible channel loaded in a first pumping segment to a common delivery conduit while filling a second compressible channel loaded in a second pumping segment with a second fluid, and to deliver the second fluid from second compressible channel to the common delivery conduit while filling the first compressible channel using the first pumping segment.

2. The infusion system of claim 1, wherein the multiple pumping segments comprise a first pumping segment and a second pumping segment, the pumping elements of each of the first and second pumping segments comprising an upstream occluder, a downstream occluder, and a plunger, wherein delivering a fluid from a respective compressible channel comprises:
filling the respective compressible channel while the upstream occluder is open and the downstream occluder is closed, closing the upstream occluder and opening the downstream occluder after the respective compressible channel is filled, and compressing the respective compressible channel using the plunger while the downstream occluder is opened,
wherein the processor is further configured to:
open the upstream occluder of the first pumping segment while the upstream occluder of the second pumping segment is closed or closing; and
close the upstream occluder of the first pumping segment while the upstream occluder of the second pumping segment is open or opening.

3. The infusion system of one of the previous claims, wherein the infusion device includes a pump housing and the multiple pumping segments are retained by the pump housing of the infusion device, wherein the pump housing is configured to simultaneously receive the first and second compressible channels.

4. The infusion system of one of the previous claims, wherein the infusion device comprises:
a first cam system including one or more cams for operating the pumping elements of the first pumping segment; and
a second cam system including one or more cams for operating the pumping elements of the second pumping segment.

5. The infusion system of one of the previous claims, wherein each upstream occluder, each downstream occluder, and each plunger of the first and second pumping segments are joined together by respective levers to coordinate movement of the first and second pumping segments as a respective pumping element set, a respective lever coordinating a motion of each pumping element of the respective pumping element set based on a motion of the other pumping element of the respective pumping element set.

6. The infusion system of any one of one of the previous claims, wherein the timing pattern for operating the first pumping segment is offset from the timing pattern for operating the second pumping segment based on a rotation of a first cam system being rotationally offset from a rotation of a second cam system by a predetermined number of degrees greater than zero.

7. The infusion system of any one of one of the previous claims, wherein the infusion device comprises a control unit and first and second pump modules removably connected to the control unit by way of respective plugin ports on the control unit, the first pump module comprising the first pumping segment and the second pump module comprising the second pumping segment, wherein the control unit comprises the processor and the processor communicates with the first and second pump modules via electrical signals communicated through the respective plugin ports.

8. The infusion system of any one of one of the previous claims, further comprising:
a dual channel infusion set comprising the first compressible channel and the second compressible channel each joining together at a downstream junction for fluidic communication with the common delivery conduit, wherein the first compressible channel and the second compressible channel each include an upstream connector for connecting to a different fluid source.

9. The infusion system of any one of one of the previous claims, wherein the common delivery conduit is an intravenous tubing connected to a patient.

10. The infusion system of one of the previous claims, wherein operating each group of serially aligned pumping elements according to respective timing patterns comprises operating the groups of serially aligned pumping elements so that the respective deliveries of the first and second fluids are continuous but do not overlap.

11. A machine-implemented method, comprising:
receiving a first compressible channel into a first pumping segment of an infusion device, the first pumping segment comprising a first group of serially-aligned pumping elements that operate collectively to delivery a first fluid from a first compressible channel when the first compressible channel is received into the first pumping segment;
receiving, while the first compressible channel is received into the first pumping segment, a second compressible channel into a second pumping segment of the infusion device, the second pumping segment comprising a second group of serially-aligned pumping elements that operate collectively to delivery a second fluid from a second compressible channel when the second compressible channel is received into the second pumping segment; and
operating each group of serially-aligned pumping elements of the first and second pumping segments according to respective timing patterns offset from each other to deliver the first fluid from the first compressible channel received in the first pumping segment to a common delivery conduit while filling the second compressible channel loaded in the second pumping segment with the second fluid, and to deliver the second fluid from second compressible channel to the common delivery conduit while filling the first compressible channel using the first pumping segment.

12. The machine-implemented method of claim 11, wherein each of the first and second pumping segments comprise an upstream occluder, a downstream occluder, and a plunger, wherein delivering a fluid from a respective compressible channel comprises:
filling the respective compressible channel while the upstream occluder is open and the downstream occluder is closed, closing the upstream occluder and opening the downstream occluder after the respective compressible channel is filled, and compressing the respective compressible channel using the plunger while the downstream occluder is opened,
wherein the machine-implemented method further comprises:
opening the upstream occluder of the first pumping segment while the upstream occluder of the second pumping segment is closed or closing; and
closing the upstream occluder of the first pumping segment while the upstream occluder of the second pumping segment is open or opening.

13. The machine-implemented method of one of claims 11 or 12, wherein the timing pattern for operating the first pumping segment is offset from the timing pattern for operating the second pumping segment based on a rotation of a first cam system being rotationally offset from a rotation of a second cam system by a predetermined number of degrees greater than zero.

14. The machine-implemented method of any one of claims 11 through 13, wherein each upstream occluder, each downstream occluder, and each plunger of the first and second pumping segments are joined together by respective levers to coordinate movement of the first and second pumping segments as a respective pumping element set, the method further comprising:
a respective lever coordinating a motion of each pumping element of the respective pumping element set based on a motion of the other pumping element of the respective pumping element set.

15. An infusion pump, comprising:
first and second pumping segments configured to operate in parallel with each other, each pumping segment including a group of serially-aligned pumping elements configured to compress an elongated compressible channel loaded within the pumping segment; and
a processor configured to, when a respective compressible channel is loaded in each of the first and second pumping segments:
cause each group of serially-aligned pumping elements of the first and second pumping segments to pump according to respective timing patterns offset from each other to deliver a first fluid from a first compressible channel loaded in the first pumping segment to a common delivery conduit while filling a second compressible channel loaded in the second pumping segment with a second fluid, and to deliver the second fluid from second compressible channel to the common delivery conduit while filling the first compressible channel using the first pumping segment.
